# EUROPÄISCHE PATENTANMELDUNG

(11) **EP 1 110 519 A1**
(43) Veröffentlichungstag der Anmeldung: **27.06.2001**
(21) Anmeldenummer: 00127642.7
(22) Anmeldetag: 16.12.2000
(51) Int. Cl.: A61F 5/58

(54) **Sprechhilfe für Stotterer**

(30) Priorität: 16.12.1999 DE 19960710; 17.02.2000 DE 10007275
(71) Anmelder: Voicetronic GmbH, 73760 Ostfildern-Kemnat (DE)
(72) Erfinder: Küppers, Bert Dr.-Ing, D-52159 Roetgen (DE)
(74) Vertreter: Jany, Peter, Dr.

(57) **Zusammenfassung**

Es wird eine für Stotterer geeignete, ein Maskierungsgeräusch erzeugende Sprechhilfe vorgeschlagen, die ein sicheres Ein-Ausschaltverhalten aufweist und eine sehr gute Eigen-Fremd-Kennung ermöglicht. Hierzu wird eine spezielle elektronische Schaltung und ein schalldichter Abschluß eines Ohres vorgeschlagen, wodurch insbesondere eine telefontaugliche Sprechhilfe realisiert wird.

## Beschreibung

Die Erfindung betrifft Sprechhilfen für Personen mit Sprachstörungen wie Stottern oder Stammeln zum Ermöglichen eines fehlerfreien oder fehlerreduzierten Sprechens. Derartige Sprechhilfen umfassen einen Sprachaufnehmer zum Aufnehmen von Sprechgeräuschen der sprachgestörten Person, einen Geräuschgeber zum Erzeugen eines Maskierungsgeräusches im Ohr der sprachgestörten Person während des Sprechens und eine elektronische Schaltung zum Erzeugen und Einschalten des Maskierungsgeräuschs während des Sprechens sowie zum Ausschalten des Maskierungsgeräuschs in Sprechpausen.

Es ist bekannt, daß Sprachstörungen wie Stottern oder Stammeln ursächlich mit dem von den Stimmbändern ausgehenden Körperschall in Verbindung stehen. Es ist ferner bekannt, daß Stotterer fehlerlos oder fehlerreduziert sprechen, wenn man das Prinzip der Maskierung anwendet. Bei der Maskierung wird während des Sprechens ein Maskierungsgeräusch über einen Schall- oder Vibrationswandler dem Gehör der sprachgestörten Person zugeführt, das eine so große Lautstärke hat, daß die sprachgestörte Person sich selbst nicht oder nur stark gedämpft sprechen hört. Die Kopplung zwischen Sprechzentrum und Sprechorgan wird unterbunden. Die Person kann dann ihre eigene Stimme, und zwar den Luft- und/oder den Körperschall, nicht selektiv wahrnehmen und dadurch fehlerfrei oder fehlerreduziert sprechen.

Damit die sprachgestörte Person einen Dialog führen kann, wird das Maskierungsgeräusch nur eingeschaltet, wenn die Person selbst spricht. Hört sie dagegen wieder auf zu sprechen, schaltet sich das Maskierungsgeräusch wieder aus. Hierzu ist eine elektronische Schaltung erforderlich, die das Maskierungsgeräusch ein- bzw. ausschaltet und einen Tongenerator zum Erzeugen des Maskierungsgeräuschs umfaßt. Ferner ist hierzu ein Sprachaufnehmer zum Aufnehmen von Sprechgeräuschen der sprachgestörten Person erforderlich, der den Beginn und das Ende des Sprechens detektiert. Hierzu können nach dem Stand der Technik Sprechaufnehmer verwendet werden, die entweder für Luftoder für Körperschall empfindlich sind, beispielsweise in Form von Kehlkopfmikrophonen oder Körperschallaufnehmern. Das Maskierungsgeräusch wird nach dem Stand der Technik mittels eines Lautsprechers als Luftschall erzeugt.

Einzelheiten zu vorteilhaften Merkmalen der Maskierung, beispielsweise hinsichtlich der Maskierungslautstärke, der Frequenz oder des Frequenzbereichs des Maskierungsgeräuschs, der Art des Maskierungsgeräuschs (künstlich erzeugte Töne oder eigene Sprache), einer günstigen Einschaltschwelle und Einschaltempfindlichkeit, einer Ausschaltverzögerung, Frequenzfilterungen, Auswahl des mit dem Maskierungsgeräusch beschallten Ohres usw. sind im einschlägigen Stand der Technik beschrieben.

Aus dem Dokument DE 3018947 A1 ist eine Sprechhilfe für Stotterer bekannt, bei der mittels eines von der sprachgestörten Person getragenen Kopfhörers ein Maskierungsgeräusch erzeugt wird. Das Maskierungsgeräusch wird dabei durch Signale eines am Kehlkopf getragenen Kehlkopfmikrophons ausgelöst. Zum Führen eines Dialoges werden Fremdgeräusche mittels eines Luftschallmikrophons aufgenommen und ebenfalls über den Kopfhörer auf das Ohr übertragen.

Das Dokument DE 3146556 A1 offenbart eine Sprechhilfe für Stotterer, bei der mittels eines Kehlkopfmikrophons der Beginn des Sprechens erfaßt und die Sprache verzögert und mittels eines Tiefpasses frequenzgefiltert über einen Telefonhörer dem Ohr des Stotterers zugeführt wird.

In dem Dokument DE 3303418 A1 ist eine Sprechhilfe für Stotterer beschrieben, bei der mittels eines Luftschallaufnehmers ein Maskierungsgeräusch ausgelöst wird.

Das Dokument DE 3506092 A1 zeigt eine Sprechhilfe für Stotterer, mittels der ein zeitverzögertes Signal unter Verwendung eines Kopfhörers oder einer Otoplastik dem Ohr zugeführt wird. Weitere dort beschriebene Ausführungsformen betreffen Kinnbügelhörer, Halsbänder und Brillengestelle. Das Sprechgeräusch wird entweder mittels eines in der Nähe des Mundes angeordneten Luftschallmikrophons oder mittels eines am Kehlkopf getragenen Kehlkopfmikrophons aufgenommen.

Im dem Dokument DE 3541117 A1 ist eine Sprechhilfe für Stotterer beschrieben, bei der Luft- oder Körperschallaufnehmer im Ohr angeordnet sind und ein im Ohr angeordneter Wandler sowohl als Schallaufnehmer als auch als Maskierungssignalgeber dienen kann.

Das Dokument DE 3922607 A1 zeigt eine Sprechhilfe für Stotterer in Form einer Brille. In dem Brillengestell ist ein Mikrophon als Sprechaufnehmer angeordnet. Die aufgenommene Sprache wird mittels eines akustischen Wandlers dem Ohr zugeführt.

Die aus dem Stand der Technik bekannten Sprechhilfen weisen jedoch eine Vielzahl von Nachteilen auf. Ein Hauptproblem besteht darin, daß die bekannten Sprechhilfen auffällig sind, da es sich zumeist um große externe Geräte handelt, die beispielsweise am Gürtel getragen werden und mittels Schläuchen oder Kabeln mit dem Ohr bzw. mit Kopfhörern verbunden sind. Insbesondere auffällig sind Kopfhörer, mittels denen das Maskierungsgeräusch erzeugt wird, Kabel- oder Schlauchverbindungen am Körper zwischen dem Ohr und an einem anderen Ort angebrachten Geräte sowie Kehlkopfmikrophone einschließlich ihrer Zuleitung.

Dadurch ist für eine dritte Person sofort erkennbar, daß der Träger einer solch auffälligen Sprechhilfe eine Besonderheit aufweist. Durch den optischen und ästhetischen Eindruck wird der Anschein einer bestehenden Krankheit erweckt und die Kontaktpflege wird beeinträchtigt. Da ferner die bekannten Sprechhilfen keinen guten Tragekomfort bieten, aufwendig zu montieren sind oder in der bekannten Ausführungsform als Brille auch Personen, die keine Sehbrille benötigen, ein Brillengestell tragen müssen, ist die Akzeptanz sprachgestörter Personen, derartige Sprechhilfen zu verwenden, trotz der mit ihnen verbundenen Vorteile in Bezug auf die Verbesserung der Sprachstörung weitgehend gering.

Dies ist auch darauf zurückzuführen, daß bisher keine elektronischen Schaltungen entwickelt werden konnten, die es ermöglichen, derartige Sprechhilfen in miniaturisierter Form wenig auffällig als In-dem-Ohr- oder Hinter-dem-Ohr-Geräte zu realisieren, die ohne großvolumige Spannungsversorgungen auskommen und auf der im Hörgerätebereich bekannten, mit Knopfzellen realisierten 1,4 Volt-Technik basieren.

Ein weiteres Problem bekannter Sprechhifen besteht in ihrer oft unzureichenden Zuverlässigkeit beim Ein- und Ausschalten des Maskierungsgeräusch zum jeweils erforderlichen Zeitpunkt. Dieses Problem bezieht sich genau genommen auf zwei unterschiedliche Aspekte. Der eine Aspekt betrifft die Tatsache, daß eine Mit- bzw. Rückkopplung zwischen dem von dem Geräuschgeber abgegebenen Maskierungsgeräusch und dem von dem Sprachaufnehmer aufgenommenen Sprechgeräusch verhindert werden muß, damit sich das Maskierungsgeräusch wieder abschalten läßt und nicht permanent eingeschaltet bleibt. Der andere Aspekt betrifft eine zuverlässige Unterscheidung zwischen Eigenschall der sprachgestörten Person und Fremdschall von anderen Personen oder anderen Schallquellen, damit das Markierungsgeräusch zuverlässig nur dann ausgelöst wird, wenn die sprachgestörte Person selbst spricht, und nicht ausgelöst wird, wenn externer Schall wahrgenommen wird. Dies ist insbesondere in halligen Räumen, beispielsweise Klassenzimmern, oder beim Telefonieren schwierig zu realisieren.

Ferner sind die bekannten Sprechhilfen oft gesundheitlich bedenklich, da sie Maskierungsgeräusche mit hohen Lautstärken erzeugen, die zu Ohrschädigungen führen können.

Der Erfindung liegt unter Berücksichtigung dieses Standes der Technik die Aufgabe zugrunde, eine Sprechhilfe für Personen mit Sprachstörungen zu schaffen, die unauffälliger zu tragen ist, einen besseren Tragekomfort bietet und ohne erheblichen Aufwand am Körper befestigt und ohne besondere Umstände eingesetzt werden kann, um dadurch die Akzeptanz der betroffenen Personen, solche Sprechhilfen zu verwenden, zu erhöhen. Weiterhin ist es wünschenswert, die Zuverlässigkeit von Sprechhilfen hinsichtlich ihres Ein- und Ausschaltverhaltens zu verbessern, auch um mit ihnen verbundene gesundheitliche Gefahren zu verringern, sowie eine bessere Eigen-Fremd-Kennung zu erzielen, um sie beispielsweise auch in halligen Räumen oder beim Telefonieren verwenden zu können.

Diese Aufgabe wird erfindungsgemäß durch eine Sprechhilfe mit den Merkmalen des beigefügten Anspruchs 1 gelöst.

Bevorzugte Ausgestaltungen und Weiterbildungen der Erfindung ergeben sich aus den Ansprüchen 2-25 und der nachfolgenden Beschreibung mit zugehörigen Zeichnungen. Die darin beschriebenen Besonderheiten können einzeln oder in Kombination miteinander eingesetzt werden, um bevorzugte Ausgestaltungen der Erfindung zu schaffen, wobei sich durch das Zusammenwirken von Merkmalen zusätzliche vorteilhafte Wirkungen ergeben können.

Eine erfindungsgemäße Sprechhilfe für Personen mit Sprachstörungen wie Stottern oder Stammeln zum Ermöglichen eines fehlerfreien oder fehlerreduzierten Sprechens umfaßt also einen Sprachaufnehmer zum Aufnehmen von Sprechgeräuschen der sprachgestörten Person, einen Geräuschgeber zum Erzeugen eines Maskierungsgeräuschs im Ohr der sprachgestörten Person während des Sprechens und eine elektronische Schaltung zum Erzeugen und Einschalten des Maskierungsgeräuschs während des Sprechens sowie zum Ausschalten des Maskierungsgeräuschs in Sprechpausen. Sie weist die Besonderheit auf, daß die elektronische Schaltung eine Triggerschaltung zum Ein- und Ausschalten des Maskierungsgeräuschs umfaßt, die derart ausgebildet ist, daß das Maskierungsgeräusch nach dem Einschalten durch ein Einschaltsignal des Sprachaufnehmers während einer vorgegebenen Einschaltdauer aufrechterhalten wird, nach Ablauf der Einschaltdauer das Maskierungsgeräusch abgeschaltet wird und nach dem Ende der Einschaltdauer erst nach Ablauf einer vorgegebenen Pausendauer wieder durch ein Einschaltsignal des Sprachaufnehmers eingeschaltet werden kann. Dieser Vorgang wiederholt sich zyklisch, solange der Träger spricht.

Durch diese erfindungsgemäßen Merkmale wird eine Mitkopplung zwischen dem Geräuschgeber und dem Sprachaufnehmer wirksam unterbunden, insbesondere wenn ein elektrodynamischer Wandler verwendet wird, der sowohl als Geräuschgeber als auch als Sprachaufnehmer ausgebildet ist. Während der Einschaltdauer wird das Maskierungsgeräusch erzeugt, und nach dem Ende der Einschaltdauer wird während der Pausendauer ein Abklingen des Geräuschgebers und ein Abklingen des von ihm erzeugten Schalls ermöglicht, bevor der Sprachaufnehmer wieder mit hoher Empfindlichkeit beim Detektieren des Sprechens des Trägers das Maskierungsgeräusch einschalten kann.

Die Pausendauer wird dabei vorteilhafterweise so kurz gewählt, daß der Träger der Sprechhilfe, falls er am Ende einer Einschaltdauer noch spricht, nicht durch die Unterbrechung des Maskierungsgeräuschs während der Pausendauer irritiert wird. Wenn der Träger noch spricht, setzt das Maskierungsgeräusch nach der kurzen Pausendauer wieder ein, ohne daß dies der Träger bewußt wahrnimmt oder er zumindest nicht durch die Unterbrechung zu stottern beginnt. Wenn dagegen der Träger am Ende einer Pausendauer nicht spricht, bleibt das Maskierungsgeräusch ausgeschaltet, bis ein neues Einschaltsignal von dem Sprachaufnehmer dies verursacht.

Nach einem zusätzlichen vorteilhaften Merkmal wird vorgeschlagen, daß die Einschaltdauer größer als die Pausendauer, vorzugsweise zwischen zwei- und zehnmal so groß wie die Pausendauer ist. Nach einem anderen vorteilhaften Merkmal kann vorgesehen sein, daß die Einschaltdauer zwischen 0,1 und 2,0 s, bevorzugt zwischen 0,3 und 0,7 s beträgt. Die Pausendauer wird vorteilhafterweise zwischen 0,02 und 0,5 s, bevorzugt zwischen 0,05 und 0,2 s betragen.

Die Einschaltdauer und die Pausendauer können mittels an sich bekannter elektronischer Logikgatter realisiert werden. Dabei kann beispielsweise die Pausendauer mittels eines Logikgatters durch eine elektronische Austastung oder eine Deaktivierung des Einschaltsignals des Sprachaufnehmers oder des Eingangs der Triggerschaltung während der Pausendauer realisiert sein. Derartige Schaltungen sind jedoch relativ groß und können nach dem bekannten Stand der Technik nicht mit 1,4 Volt-Technik miniaturisiert hergestellt werden.

Aus diesen Gründen ist es bevorzugt, wenn die Einschaltdauer und auch die Pausendauer mittels einer analogen Schaltung realisiert sind. Die Pausendauer kann vorteilhafterweise mittels einer analogen Schaltung durch eine Anhebung der Triggerschwelle der Triggerschaltung gegenüber dem Einschaltsignal des Sprachaufnehmers realisiert sein.

Bei erfindungsgemäßen Sprechhilfen können als Sprachaufnehmer sowohl akustische, Umgebungsschall aus der Luft aufnehmende Mikrophone als auch mechanische Körperschallschwingungen von Knochen aufnehmende Vibrationssensoren verwendet werden. Ein Sprachaufnehmer ist bevorzugt in der Nähe des Trommelfells angeordnet. Dies ist eine günstige Stelle, um die durch die Eustachische Röhre übertragenen akustischen Schwingungen vom Knochen aufzunehmen. Der Vibrationssensor ist vorteilhafterweise zwischen dem ersten und zweiten Knick im Gehörgang, vorzugsweise in einem Abstand von 5 bis 10 mm vom Trommelfell angeordnet. Dort ist die Gehörgangshaut am dünnsten. Der Vibrationssensor steht vorzugsweise im Hautkontakt zu der Gehörgangswand, um eine Übertragung der mechanischen Schwingungen von Schädelknochen und der Gehörgangswand auf den Vibrationssensor zu ermöglichen. Er kann mittels geeigneter elastischer Materialien an die Gehörgangswand angedrückt werden.

Nach einem anderen vorteilhaften Merkmal wird vorgeschlagen, daß der Vibrationssensor im Bereich des Ohrläppchens zwischen dem Schläfenbein und dem Unterkiefer, vorzugsweise auf den Unterkiefer gerichtet, angeordnet ist. In diesem Bereich bildet sich eine Vertiefung oder Mulde aus, die spürbar die Nahtstelle dieser beiden Knochen darstellt. An dieser Stelle sind die Körperschallvibrationen deutlich zu spüren, so daß sie besonders geeignet ist, um während des Sprechens das Einschalten des Maskierungsgeräuchs auszulösen.

Gemäß einer weiteren, vorteilhaften Ausführungsform kann vorgesehen sein, daß die Sprechhilfe einen Sprachaufnehmer aufweist, der ein sowohl für akustische Schallwellen als auch für Vibrationen empfindlicher Sensor ist. Auf diese Weise kann erreicht werden, daß beide Schallanteile gleichermaßen detektiert werden.

Eine andere vorteilhafte Ausführungsform kann sein, daß die Sprechhilfe sowohl ein akustisches Mikrophon als auch einen Vibrationssensor aufweist, und die elektronische Schaltung derart ausgebildet ist, daß sie nur dann das Maskierungsgeräusch auslöst, wenn gleichzeitig von den beiden Sprachaufnehmern ein Sprechgeräusch aufgenommen wird. Wenn zwei verschiedene Sprachaufnehmer verwendet werden, deren koinzidentes Signal zum Einschalten des Maskierungsgeräuschs verwendet wird, wird das Einschaltverhalten verbessert. Es kommt dann erheblich weniger zu Fehleinschaltungen des Maskierungsgeräuschs und die Funktionssicherheit der Sprechhilfe ist erhöht.

Der das Maskierungsgeräusch erzeugende Geräuschgeber kann vorteilhafterweise einen akustischen Schall erzeugenden Lautsprecher umfassen. Eine andere vorteilhafte Ausbildung eines das Maskierungsgeräusch erzeugenden Geräuschgebers kann darin bestehen, daß dieser einen Vibrationsgeber zum Erzeugen von in Knochen eingeleiteten mechanischen Vibrationen umfaßt. Dies kann zweckmäßig sein, um von Maskierungsgeräuschen ausgehende Gehörschäden zu vermeiden oder zu verringern. Mit einem anstatt oder zusätzlich zu einem akustischen Geräuschgeber verwendeten Vibrationsgeber kann die akustische Belastung des Ohres mit dem Maskierungsgeräusch, d.h. der Schalldruck auf dem Trommelfell stark vermindert werden, da das Maskierungsgeräusch zumindest teilweise mit dem Vibrationsgeber erzeugt wird. Dadurch wird der weitere Vorteil erzielt, daß sich das Ohr der sprachgestören Person nach dem Sprechen schneller regeneriert, d.h. sich die Hörempfindlichkeit für das Hören von Umgebungsgeräuschen schneller wieder erhöht.

Der Vibrationsgeber kann mechanische Vibrationen erzeugen, die von der Sprache der sprachgestörten Person abgeleitet sind, d.h. in ihrem Intensitäts - und/oder Zeitverlauf der Sprache entsprechen. Besonders einfach zu realisieren ist eine Ausbildung, bei der der Vibrationsgeber unabhängig von dem Sprechrythmus eine oder mehrere vorgegebene Schwingungen erzeugt.

In manchen Ausführungsformen kann es zweckmäßig sein, wenn sie sowohl einen Lautsprecher als auch einen Vibrationsgeber umfassen, um auf diese Weise sowohl akustischen Schall als auch mechanischen Körperschall als Maskierungsgeräusch zu erzeugen.

Nach einem besonders bevorzugten Merkmal wird vorgeschlagen, daß die Sprechhilfe einen elektrodynamischen Wandler umfaßt, der sowohl als Geräuschgeber als auch als Sprachaufnehmer ausgebildet ist. Eine solche Ausführungsform ist nicht nur besonders kostengünstig herstellbar, da entweder ein Geräuschgeber oder ein Sprachaufnehmer eingespart wird, sondern sie hat auch den besonderen Vorteil, daß sie besonders klein ausgebildet sein kann, so daß sie unauffällig am äußeren Ohr oder sogar im Gehörgang angeordnet werden kann.

Bevorzugt ist vorgesehen, daß die Sprechhilfe ein in ein Ohr einsetzbares Ohrstück umfaßt, das einen Sprachaufnehmer umfaßt. Vorteilhafterweise kann darüber hinaus vorgesehen sein, daß das Ohrstück auch einen Geräuschgeber aufweist.

Ein solches Ohrstück kann eine individuell paßgenau angefertigte, in den Gehörgang der sprachgestörten Person einsetzbare Otoplastik in Form einer In-dem-Ohr-Schale (im folgenden IO-Schale) umfassen, in die der Sprechaufnehmer, der Geräuschgeber und ggf. die elektronische Schaltung integriert sind. Es befinden sich also alle Komponenten der Sprechhilfe vollständig zusammengefaßt im Ohr, was beim Tragen eine außerordentliche Erleichterung darstellt. Die Sprechhilfe ist dadurch sehr unauffällig und erweckt, sofern sie überhaupt von einem Außenstehenden wahrgenommen wird, den Anschein, als würde der Träger ein Hörgerät im Ohr haben. Das Gerät ist aber nicht nur wie ein im Ohr getragenes Hörgerät sehr unauffällig, sondern auch sehr klein und hat ein geringes Gewicht. Der Tragekomfort ist hoch und es ist unmittelbar und schnell ohne großen Aufwand im Ohr zu befestigen.

Eine IO-Schale ist vorzugsweise als dünne, harte Ohrschale ausgebildet, die einen Hohlraum zur Aufnahme des Sprechaufnehmers, des Geräuschgebers und der elektronischen Schaltung aufweist. Sie kann entsprechend einer Im-Ohr-Plastik eines Hörgerätes hergestellt werden. Einzelheiten zu geeigneten Materialien und Herstellungsverfahren sind der Literatur, beispielsweise dem Werk von Ulrich Voogdt, "Otoplastik", Median-Verlag 1993 zu entnehmen. Von besonderem Vorteil ist, wenn die IO-Schale nach einem Ohrabdruck von dem Ohrkanal angefertigt wird. Eine vorteilhafte Besonderheit kann darin bestehen, daß in der IO-Schale eine Öffnung oder Nut vorhanden ist, in der ein Sprachaufnehmer angeordnet ist, der als mechanische Körperschallschwingungen von Knochen aufnehmender Vibrationssensor ausgebildet ist und an die Gehörgangswand gedrückt wird.

Nach einem anderen vorteilhaften Merkmal kann vorgesehen sein, daß die Sprechhilfe eine vorzugsweise individuell paßgenau angefertigte, in den Gehörgang der sprachgestörten Person einsetzbare Otoplastik in Form eines Ohrstücks für Hinter-dem-Ohr-Geräte umfaßt, in das der Sprachaufnehmer integriert ist und das zum Zuführen des von dem Geräuschgeber erzeugten Maskierungsgeräuschs zu dem Gehörgang vor dem Trommelfell dient, und ein auf das Ohr aufsetzbares Tragteil, von dem die elektronische Schaltung getragen wird oder in das die elektronische Schaltung integriert ist. Das Tragteil kann auch mit der Batterie und den erforderlichen Einstellreglern versehen sein.

Auch bei dieser Ausführungsform ist der Tragekomfort gut, wobei bei entsprechender Ausgestaltung die Sprechhilfe unauffällig sein kann. Im Hinblick darauf, daß sie aber auch Elemente außerhalb des Ohres aufweist, werden die Vorteile der oben beschriebenen Sprechhilfe nicht ganz erreicht.

Das Ohrstück ist dabei vorzugsweise im wesentlichen massiv ausgebildet, d.h. ohne größere Hohlräume. Ferner ist es, ebenfalls im Gegensatz zu der oben beschriebenen IO-Schale weichelastisch ausgebildet, beispielsweise silikonartig. Der Sprachaufnehmer kann in eine entsprechend angeordnete Bohrung geklebt sein.

Das Ohrstück sollte nicht zu fest im Ohr sitzen, da sonst beim Anwender Druckstellen und Schmerzen verursacht werden können. Es muß hierbei eine ausgewogene und für den Anwender und die Funktion optimale Anpassung individuell angestrebt werden. Bei einem weichen, silikonartigen Ohrstück wird es weniger zu Druckstellen als bei einem harten Ohrstück kommen, wobei weiterhin vorteilhaft ist, wenn mittels einer gummiartigen Oberfläche die Haftung im Ohr verbessert ist.

Ein besonders vorteilhaftes Merkmal, daß auch bei bekannten Sprechhilfen vorteilhaft einsetzbar ist, besteht darin, daß das Ohrstück derart ausgebildet ist, daß es den Gehörgang schalldicht oder schallreduzierend abschließt, um den Sprachaufnehmer gegen Umgebungsschall abzuschirmen. Hierdurch wird eine besonders gute Eigen-Fremd-Kennung ermöglicht, so daß der Geräuschgeber nur dann eingeschaltet wird, wenn der Träger der Sprechhilfe selbst spricht. Dadurch, daß das Ohrstück den im Gehörgang angeordneten Sprachaufnehmer schalldicht oder schallreduzierend gegen Fremdschall abschirmt, reagiert der Sprachaufnehmer nicht auf Fremdschall und es kommt nicht zu Fehlauslösungen des Geräuschgebers.

Nach einem zusätzlichen vorteilhaften Merkmal kann vorgesehen sein, daß die Sprechhilfe zwei in die Ohren einsetzbare Ohrstücke umfaßt, wobei in dem ersten Ohrstück ein Sprachaufnehmer und ein Geräuschgeber und in dem zweiten Ohrstück ein weiterer Geräuschgeber angeordnet sind. Vorteilhafterweise ist dabei zusätzlich vorgesehen, daß in dem zweiten Ohrstück kein Geräuschaufnehmer angeordnet ist. Darüber hinaus kann vorteilhafterweise vorgesehen sein, daß das erste Ohrstück den Gehörgang schalldicht oder schallreduzierend abschließt und das zweite Ohrstück einen den Umgebungsschall durchlassenden Kanal aufweist. Derartige Ausführungsformen sind ganz besonders geeignet, einem Träger einer erfindungsgemäßen Sprechhilfe das Telefonieren zu ermöglichen. Er hält hierzu das Telefon an dasjenige Ohr, in dem kein Sprachaufnehmer angeordnet ist, so daß durch den Fremdschall, den der Träger aus dem Telefonhörer wahrnimmt, das Maskierungsgeräusch nicht ausgelöst wird. Wenn dagegen der Träger selbst spricht, wird durch den in dem anderen Ohr angeordneten Sprachaufnehmer das Maskierungsgeräusch ausgelöst, so daß er fehlerfrei sprechen kann.

Eine erfindungsgemäße Sprechhilfe kann vorteilhafterweise nur einen Sprachaufnehmer umfassen. Es kann jedoch, insbesondere bei therapieresistenten Personen vorteilhaft sein, wenn sie zwei Geräuschgeber umfaßt, die in je einem Ohr eines Benutzers angeordnet werden können.

Mit der Erfindung werden somit Ziele erreicht, um die die Fachwelt sich schon lange bemüht hat. Die Erfindung wird nachfolgend anhand in den Figuren dargestellter Ausführungsbeispiele näher erläutert. Es zeigen:
- Fig. 1: eine erfindungsgemäße elektronische Schaltung,
- Fig. 2: zwei parallel geschaltete elektrodynamische Wandler,
- Fig. 3: eine Abwandlung zu Fig. 1,
- Fig. 4: eine zweite Abwandlung zu Fig. 1 und
- Fig. 5: eine erfindungsgemäße Sprechhilfe.

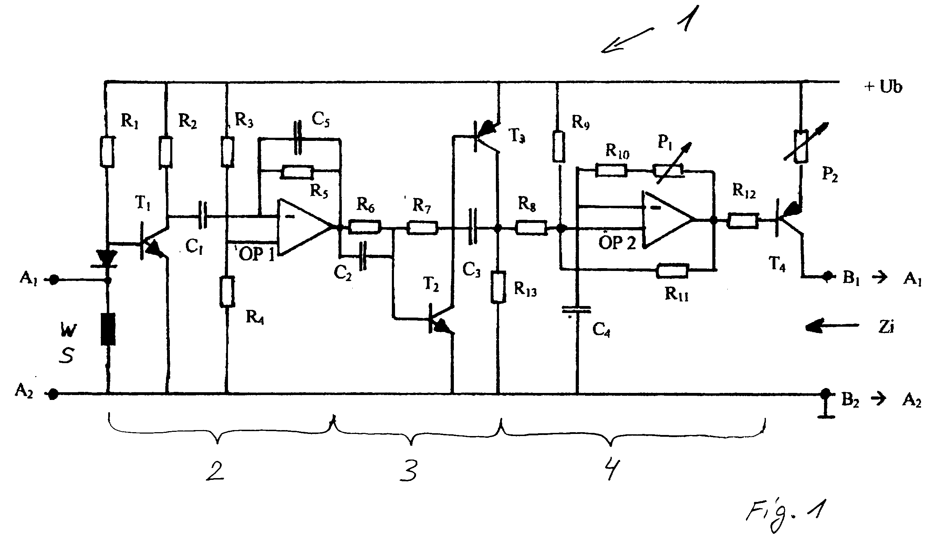

Die Fig. 1 zeigt eine elektronische Schaltung 1 zum Ein-und Ausschalten eines Maskierungsgeräuschs. Sie umfaßt einen Eingang A₁, A₂, an den ein Sprachaufnehmer S angeschlossen ist. Der Sprachaufnehmer S kann akustischen Luftschall und/oder Körperschwingungen aufnehmen. Die elektronische Schaltung 1 umfaßt im wesentlichen drei Baugruppen, nämlich einen Eingangsverstärker 2, eine Triggerschaltung 3 und einen Tongenerator 4, der an dem Ausgang B₁, B₂ das Signal für einen das Maskierungsgeräusch erzeugenden Geräuschgeber bereitstellt. Der Tongenerator 4 umfaßt einen astabilen Multivibrator, der durch den Operationsverstärker OP2 gebildet wird, und einen Transistor T₄, der als Ausgangsverstärker dient. Am Ausgang B₁, B₂ wird der Geräuschgeber angeschlossen. Eine Besonderheit der elektronischen Schaltung 1 besteht darin, daß sie sich mittels aus dem Bereich der Hörgeräte bekannter Bauelemente in 1,4-Volt-Technik realisieren läßt.

Der Eingangsverstärker 2 umfaßt einen als Impedanzwandler dienenden Transistor T₁, dessen Ausgangssignal über einen Koppelkondensator C₁ einem invertierenden Operationsverstärker OP1 zugeführt wird. Beginnt der Träger einer erfindungsgemäßen Sprechhilfe, zu der die elektronische Schaltung 1 gehört, zu sprechen, so werden Vibrationen und/oder Luftschallwellen vom Sprachaufnehmer S aufgenommen und das Ausgangssignal des Eingangsverstärkers 2 am Ausgang des Operationsverstärkers OP1 als Einschaltsignal für die nachfolgenden Schaltungsteile zum Einschalten des Maskierungsgeräuschs bereitgestellt.

Die Triggerschaltung 3 dient dazu, das Ein- und Ausschalten des an dem Ausgang B₁, B₂ angeschlossenem Geräuschgebers derart zu gestalten, daß das Maskierungsgeräusch nach dem Einschalten durch ein Einschaltsignal des Sprachaufnehmers S während einer vorgegebenen Einschaltdauer aufrecht erhalten wird, nach Ablauf der Einschaltdauer das Maskierungsgeräusch abgeschaltet wird und nach dem Ende der Einschaltdauer erst nach Ablauf einer vorgegebenen Pausendauer wieder durch ein Einschaltsignal des Sprachaufnehmers S eingeschaltet werden kann.

Hierzu ist die Triggerschaltung 3 vorzugsweise derart ausgebildet, daß die Pausendauer mittels eines Schmitt-Triggers bzw. einer analogen Schaltung zur Anhebung der Triggerschwelle der Triggerschaltung 3 gegenüber dem Einschaltsignal des Sprachaufnehmers oder mittels eines monostabilen Multivibrators bzw. Univibrators oder Mono-Flops oder Flip-Flops realisiert ist. In dem in Fig. 1 dargestellten Ausführungsbeispiel ist die Pausendauer mittels einer analogen Schaltung durch eine Anhebung der Triggerschwelle der Triggerschaltung gegenüber dem Einschaltsignal des Sprachaufnehmers realisiert.

Die Triggerschaltung 3 umfaßt einen monostabilen Multivibrator, der einen stabilen Ausgangszustand aufweist, in dem er durch ein Einschaltsignal des Sprachaufnehmers S getriggert werden kann, und einen stabilen Einschaltzustand aufweist, aus dem er nach der Einschaltdauer in den Ausgangszustand zurückkehrt, wobei das erneute Triggern nach der Rückkehr in den Ausgangszustand während der Pausendauer verhindert wird.

Weitere Schaltungsbesonderheiten bestehen darin, daß der monostabile Multivibrator zwei Transistoren T₂, T₃ umfaßt, wobei an die Basis des ersten Transistors T₂ das Einschaltsignal des Sprachaufnehmers S angelegt wird, die Basis des zweiten Transistors T₃ an den Kollektor bzw. Emitter des zweiten Transistors T₃ angeschlossen ist und der Kollektor bzw. Emitter des zweiten Transistors T₃ über einen mit einem Kondensator C₃ in Reihe geschalteten Widerstand R₇ mit der Basis des ersten Transistors T₂ verbunden ist. Das Einschaltsignal des Sprachaufnehmers S wird dabei über einen parallel zu einem Kondensator C₂ geschalteten Widerstand R₆ an die Basis des ersten Transistors T₂ angelegt. Das Potential des Kollektors bzw. Emitters des zweiten Transistors T₃ dient zum Auslösen des Maskierungsgeräuschs.

Die Funktion der in Fig. 1 dargestellten Triggerschaltung 3 mit dem die Transistoren T₂ und T₃ umfassenden monostabilen Multivibrator ist wie folgt. Die Basis des Transistors C₂ erhält über den Widerstand R₆ einen Haltestrom, der durch das Ruhepotential des Eingangsverstärkers 2 bzw. OP1 und R₆ vorgegeben ist. Dadurch wird im Ausgangszustand der Transistor T₂ durchgeschaltet und der Kondensator C₃ durch den Transistor T₃ an Pluspotential +Ub gelegt.

Das elektrische Signal eines am Sprachaufnehmer S anfallenden Schallereignisses wird durch den Operationsverstärker OP1 verstärkt. An seinem Ausgang wird das Einschaltsignal erzeugt, dessen negative Amplitude den Haltestrom über den Kondensator C₂ unterbricht. Dabei wird der Kondensator C₃ durch R₁₃ auf Null-Potential gezogen und die Spannung an der Basis von T₂ negativ. In der Folge entlädt sich der Kondensator C₃ mit der Zeitkonstanten T_{ON} = C₃ (R₆ + R₇) über die Widerstände R₆ und R₇. Während dieser Zeit wird ein astabiler Multivibrator, der den Operationsverstärker OP2 umfaßt, über den Widerstand R₈ aktiviert, der über den Transistor T₄ das Maskierungsgeräusch an einen an den Ausgang B₁, B₂ angeschlossenen Geräuschgeber abgibt.

Die Zeitkonstante T_{ON} entspricht der Einschaltdauer, während der das Maskierungsgeräusch aufrechterhalten bleibt. Nach Ablauf der Einschaltdauer T_{ON} wird der Transistor T₂ wieder durchgeschaltet und die Triggerschaltung 3 kehrt von dem Einschaltzustand in den Ausgangszustand zurück, wodurch der astabile Multivibrator, d.h. der Tongenerator 4 abgeschaltet wird. Der Kondensator C₃ wird mit der Zeitkonstanten T_{OFF} = C₃ R₇ über die Basis-Emitter-Strecke von T₂ und den Widerstand R₇ wieder aufgeladen. In dieser Zeit wird ein Retriggern des monostabilen Multivibrators über R₆ und C₂ verhindert, da die Triggerschwelle gegenüber dem Potential des Auslösesignals verschoben ist. Dadurch wird eine Mittkopplung zwischen Sprachaufnehmer S und Geräuschgeber unterbunden.

Um eine sichere Funktion des monostabilen Multivibrators zu erreichen und ein vorzeitiges Retriggern zu verhindern, enthält die elektronische Schaltung 1 eine Schaltung zur Amplitudenbegrenzung des Einschaltsignals des Sprachaufnehmers S. Die Amplitude des Einschaltsignals wird durch Einstellung des Ruheausgangspotentials am Operationsverstärker OP1 durch die Widerstände R₃ und R₄ sowie durch die Begrenzung der Amplitude aufgrund des dadurch festgelegten Aussteuerbereichs des Operationsverstärkers OP1 erreicht. Dadurch wird die Amplitude des Einschaltsignals begrenzt, so daß auch bei hohen Signalen des Sprachaufnehmers S erst nach Ablauf der durch T_{OFF} vorgegebenen Zeit der monostabile Multivibrator durch ein erneutes Schallereignis wieder aktivierbar ist.

Eine weitere Besonderheit der in Fig. 1 dargestellten elektronischen Schaltung besteht darin, daß sie derart ausgebildet ist, daß der an den Ausgang B₁, B₂ angeschlossene Geräuschgeber von Gleich- und/oder Wechselspannungssignalen, die im Betriebszustand als Geräuschgeber an ihn angelegt werden, im wesentlichen freigeschaltet wird, wenn kein Maskierungsgeräusch erzeugt wird. Dies ist besonders dann vorteilhaft, wenn in der erfindungsgemäßen Sprechhilfe ein elektrodynamischer Wandler verwendet wird, der sowohl als Geräuschgeber als auch als Sprachaufnehmer dient. In Fig. 1 ist das am Ausgang B₁, B₂ dadurch veranschaulicht, daß dieser mit dem an dem Eingang A₁, A₂ angeschlossenen Sprachaufnehmer S, der gleichzeitig auch als Geräuschgeber dient, verbunden ist.

In diesem Fall ist es zweckmäßig, wenn der elektrodynamische Wandler W, der den Sprachaufnehmer S bzw. den Geräuschgeber bildet, in dem Betriebszustand als Sprachaufnehmer von Gleich- und/oder Wechselspannungssignalen, die im Betriebszustand als Geräuschgeber an den elektrodynamischen Wandler angelegt werden, im wesentlichen freigeschaltet wird. In Fig. 1 erfolgt das Freischalten des elektrodynamischen Wandlers W mittels eines in seine Zuleitung geschalteten Transistors T₄, der in dem Betriebszustand des elektrodynamischen Wandlers W als Geräuschgeber von der Triggerschaltung 3 bzw. dem das Maskierungsgeräusch erzeugenden Tongenerator 4 durchgeschaltet wird und in dem Betriebszustand des elektrodynamischen Wandlers W als Sprachaufnehmer S gesperrt ist, so daß im Betriebszustand als Sprachaufnehmer der Ausgangswiderstand Zᵢ des Ausgangs B₁, B₂ so hoch ist, daß die Empfindlichkeit des elektrodynamischen Wandlers in seiner Funktion als Sprachaufnehmer S nicht beeinträchtigt wird.

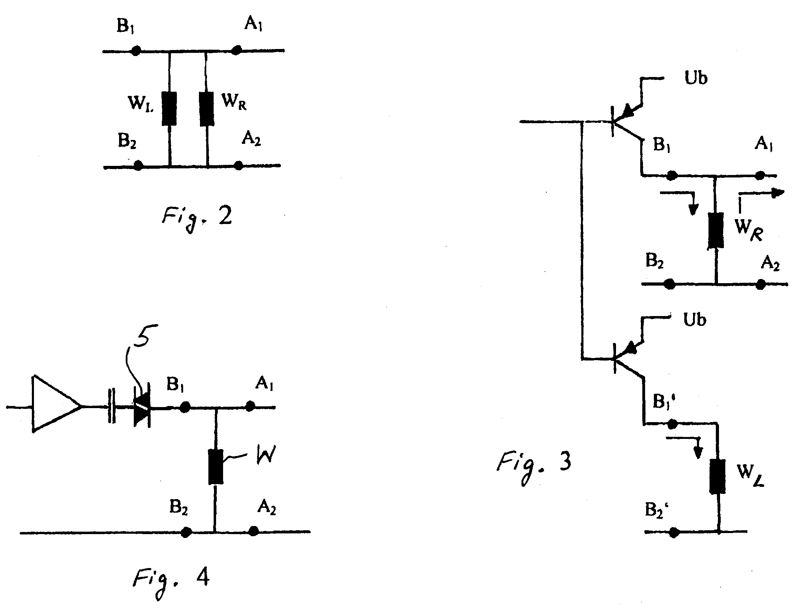

Alternativ kann vorgesehen sein, daß das Freischalten des elektrodynamischen Wandlers W in dem Betriebszustand als Sprachaufnehmer S mittels einer oder mittels zweier antiparallel in seine Zuleitung geschalteter Dioden 5 erfolgt, die in dem Betriebszustand als Geräuschgeber leitend ist bzw. sind und die in dem Betriebszustand als Sprachaufnehmer S gesperrt ist bzw. sind. In Fig. 4 ist dies schematisch mit einem elektrodynamischen Wandler W, der sowohl als Sprachaufnehmer an den Eingang A₁, A₂ als auch als Geräuschgeber an den Ausgang B₁, B₂ angeschlossen ist, veranschaulicht.

In Ausführungsformen einer erfindungsgemäßen Sprechhilfe, bei der in jedem der beiden Ohren ein Geräuschgeber angeordnet wird, können ebenfalls elektrodynamische Wandler verwendet werden, die sowohl als Sprachaufnehmer als auch als Geräuschgeber dienen. Diese Wandler können unabhängig voneinander mit jeweils einer elektronischen Schaltung arbeiten. Es kann aber auch vorgesehen sein, daß zur Synchronisation des Maskierungsgeräuschs die Wandler der beiden Ohren gemeinsam an ein Steuergerät angeschlossen sind. In Fig. 2 ist eine Parallelschaltung eines Wandlers W_{L} für das linke Ohr und eines Wandlers W_{R} für das rechte Ohr in Parallelschaltung veranschaulicht. Natürlich können die Wandler W_{L}, W_{R} auch in Reihe geschaltet sein.

Die Fig. 3 zeigt eine besonders vorteilhafte Ausführungsform mit zwei elektrodynamischen Wandlern W_{L}, W_{R}, die durch ein gemeinsames Steuergerät gesteuert werden, die in besonderem Maße geeignet ist, dem Träger der Sprechhilfe das Telefonieren zu ermöglichen. Hierzu ist in dem einen Gehörgang, angenommen dem linken Ohr, ein Wandler W_{L} untergebracht, der sowohl als Sprachaufnehmer als auch als Geräuschgeber dient. Er ist daher, wie in Fig. 3 veranschaulicht ist, in seiner Funktion als Sprachaufnehmer sowohl an den Eingang als A₁, A₂ der elektronischen Schaltung als auch in seiner Funktion als Geräuschgeber an den Ausgang B₁, B₂ der elektronischen Schaltung angeschlossen. Der Wandler W_{R} in dem anderen, angenommen dem rechten Ohr dient dagegen nur als Geräuschgeber und hat keine Funktion als Sprachaufnehmer. Er ist daher nur an einen anderen Ausgang B'₁, B'₂ der elektronischen Schaltung angeschlossen, so daß die elektronische Schaltung nicht durch Schallereignisse aus einem an dem rechten Ohr anliegenden Telefonhörer getriggert werden kann und der Schall aus dem Telefonhörer das Maskierungsgeräusch nicht auslöst.

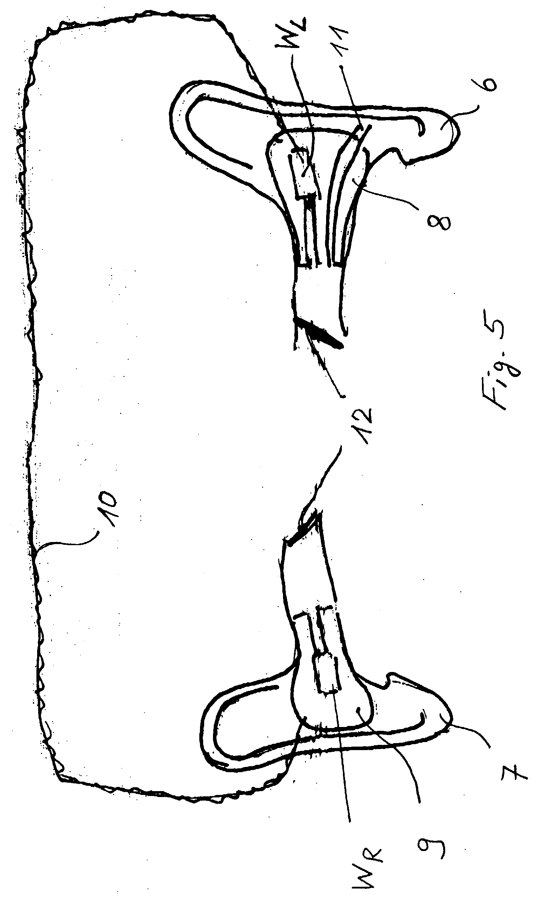

In Fig. 5 ist eine praktische Ausführungsform einer solchen telefontauglichen Sprechhilfe veranschaulicht. In die Gehörgänge des linken Ohrs 6 und des rechten Ohrs 7 sind jeweils Ohrstücke 8, 9 eingesetzt, die elektrodynamische Wandler W umfassen. Die Ohrstücke 8, 9 können je nach Anwendungsfall hart- oder weichelastisch, massiv oder hohl, in Standardgrößen oder individuell angepaßt sein. Der Wandler W_{L} in dem linken Ohr dient nur als Geräuschgeber und hat keine Funktion als Sprachaufnehmer, der Wandler W_{R} in dem rechten Ohr dient sowohl als Sprachaufnehmer als auch als Geräuschgeber.

In einem der beiden Ohrstücke 8,9 kann auch die gesamte elektronische Schaltung untergebracht sein. Alternativ ist es auch möglich, anstelle der dargestellten In-dem-Ohr-Ausführungsform Varianten zu realisieren, bei denen Komponenten in einer Hinter-dem-Ohr-Technik in hinter einem oder beiden Ohren angebrachten Gehäusen enthalten sind. Die Verbindung 10 der elektrischen und elektronischen Bauteile in und/oder an dem linken Ohr 8 mit denen des rechten Ohres 9 kann drahtgebunden, vorzugsweise über eine unauffällige Ausbildung beispielsweise über einer Halskette oder eine Brille erfolgen. Die Verbindung 10 kann aber auch mittels einer Funkverbindung realisiert sein. Auf diese Weise kann die Synchronizität einer für beide Ohren vorgesehenen Sprechhilfe gewährleistet und der Austausch von Steuersignalen ohne störende und auffällige Kabelverbindung realisiert werden.

Zur besseren Unterscheidung des Eigenschalls von Fremdschall, d.h. zur Erzielung einer optimalen Eigen-Fremd-Kennung, kann eine erfindungsgemäße Sprechhilfe bzw. eine elektronische Schaltung 1 einen Tiefpaß aufweisen, da der Eigenschall gegenüber dem Fremdschall mehr Tieffrequenzanteile enthält und somit dieses Merkmal zur Verbesserung der Unterscheidung herangezogen werden kann.

Die in Fig. 5 dargestellte Sprechhilfe veranschaulicht ein weiteres Merkmal, das zur Verbesserung der Eigen-Fremd-Kennung dient. Hierzu ist das Ohrstück 9 im rechten Ohr 7 derart ausgebildet, daß es den Gehörgang schalldicht oder schallreduzierend abschließt, um den Sprachaufnehmer gegen Umgebungsschall abzuschirmen. Das rechte Ohr 7 bzw. der Sprachaufnehmer in dem rechten Ohr nehmen dadurch keinen oder nur gedämpften Umgebungsschall wahr. Damit der Träger der Sprechhilfe dennoch hören kann, weist das Ohrstück 8 im linken Ohr 6 einen den Umgebungsschall durchlassenden Kanal 11 auf. Er ist zum im wesentlichen ungedämpften Durchlassen von Umgebungsschall ausgebildet, beispielsweise in Form einer Zusatzbohrung, eines zusätzlichen Loches oder einer eingefrästen Rille. Die Öffnung 11 ermöglicht nicht nur eine ungestörte Wahrnehmung des ambienten Schallfeldes, sondern hat auch die Funktion eines Ventilationskanals. Über die Öffnung 11 kann der Träger auch fehlerfreie Telefongespräche führen.

### Bezugszeichenliste

- 1: elektronische Schaltung
- 2: Eingangsverstärker
- 3: Triggerschaltung
- 4: Tongenerator
- 5: Dioden
- 6: linkes Ohr
- 7: rechtes Ohr
- 8: linkes Ohrstück
- 9: rechtes Ohrstück
- 10: Verbindung
- 11: Kanal
- 12: Trommelfell

- A₁: Eingang
- A₂: Eingang
- B₁: Ausgang
- B₂: Ausgang
- S: Sprachaufnehmer
- T_{ON}: Einschaltdauer
- T_{OFF}: Pausendauer
- W: Wandler
- Zᵢ: Ausgangswiderstand

## Patentansprüche

1. Sprechhilfe für Personen mit Sprachstörungen wie Stottern oder Stammeln zum Ermöglichen eines fehlerfreien oder fehlerreduzierten Sprechens, umfassend einen Sprachaufnehmers zum Aufnehmen von Sprechgeräuschen der sprachgestörten Person, einen Geräuschgeber zum Erzeugen eines Maskierungsgeräuschs im Ohr der sprachgestörten Person während des Sprechens und eine elektronische Schaltung (1) zum Erzeugen und Einschalten des Maskierungsgeräuschs während des Sprechens sowie zum Ausschalten des Maskierungsgeräuschs in Sprechpausen,
dadurch gekennzeichnet, daß
die elektronische Schaltung (1) eine Triggerschaltung (3) zum Ein- und Ausschalten des Maskierungsgeräuschs umfaßt, die derart ausgebildet ist, daß das Maskierungsgeräusch nach dem Einschalten durch ein Einschaltsignal des Sprachaufnehmers (S) während einer vorgegebenen Einschaltdauer (T_{ON}) aufrechterhalten wird,
nach Ablauf der Einschaltdauer (T_{ON}) das Maskierungsgeräusch abgeschaltet wird und
nach dem Ende der Einschaltdauer (T_{ON}) erst nach Ablauf einer vorgegebenen Pausendauer (T_{OFF}) wieder durch ein Einschaltsignal des Sprachaufnehmers (S) eingeschaltet werden kann.

2. Sprechhilfe nach Anspruch 1, dadurch gekennzeichnet, daß die Einschaltdauer (T_{ON}) größer als die Pausendauer (T_{OFF}), vorzugsweise zwischen zwei- und zehnmal so groß wie die Pausendauer (T_{OFF}) ist.

3. Sprechhilfe nach einem der vorhergehenden Ansprüche, dadurch gekennzeichnet, daß die Einschaltdauer (T_{ON}) zwischen 0,1 und 2,0 s, bevorzugt zwischen 0,3 und 0,7 s beträgt.

4. Sprechhilfe nach einem der vorhergehenden Ansprüche, dadurch gekennzeichnet, daß die Pausendauer (T_{OFF}) zwischen 0,02 und 0,5 s, bevorzugt zwischen 0,05 und 0,2 s beträgt.

5. Sprechhilfe nach einem der vorhergehenden Ansprüche, dadurch gekennzeichnet, daß die Pausendauer (T_{OFF}) mittels eines Logikgatters durch eine elektronische Austastung oder eine Deaktivierung des Einschaltsignals des Sprachaufnehmers (S) oder des Eingangs der Triggerschaltung (3) während der Pausendauer (T_{OFF}) realisiert ist.

6. Sprechhilfe nach einem der vorhergehenden Ansprüche, dadurch gekennzeichnet, daß die Pausendauer (T_{OFF}) mittels eines Schmitt-Triggers realisiert ist.

7. Sprechhilfe nach einem der vorhergehenden Ansprüche, dadurch gekennzeichnet, daß die Pausendauer (T_{OFF}) mittels einer analogen Schaltung durch eine Anhebung der Triggerschwelle der Triggerschaltung (3) gegenüber dem Einschaltsignal des Sprachaufnehmers (S) realisiert ist.

8. Sprechhilfe nach einem der vorhergehenden Ansprüche, dadurch gekennzeichnet, daß die Triggerschaltung (3) einen monostabilen Multivibrator umfaßt, der einen stabilen Ausgangszustand aufweist, in dem er durch ein Einschaltsignal des Sprachaufnehmers (S) getriggert werden kann, und einen stabilen Einschaltzustand aufweist, aus dem er nach der Einschaltdauer in den Ausgangszustand zurückkehrt, wobei das erneute Triggern nach der Rückkehr in den Ausgangszustand während der Pausendauer (T_{OFF}) verhindert wird.

9. Sprechhilfe nach Anspruch 8, dadurch gekennzeichnet, daß der monostabile Multivibrator zwei Transistoren (T₂, T₃) umfaßt, wobei an die Basis des ersten Transistors (T₂) das Einschaltsignal des Sprachaufnehmers (S) angelegt wird, die Basis des zweiten Transistors (T₃) an den Kollektor bzw. Emitter des zweiten Transistors (T₃) angeschlossen ist und der Kollektor bzw. Emitter des zweiten Transistors (T₃) über einen mit einem Kondensator (C₃) in Reihe geschalteten Widerstand (R₇) mit der Basis des ersten Transistors (T₂) verbunden ist.

10. Sprechhilfe nach Anspruch 9, dadurch gekennzeichnet, daß das Einschaltsignal des Sprachaufnehmers (S) über einen parallel zu einem Kondensator (C₂) geschalteten Widerstand (R₆) an die Basis des ersten Transistors (T₂) angelegt wird.

11. Sprechhilfe nach einem der Ansprüche 9 bis 10, dadurch gekennzeichnet, daß das Potential des Kollektors bzw. Emitters des zweiten Transistors (T₃) zum Auslösen des Maskierungsgeräuschs dient.

12. Sprechhilfe nach einem der Ansprüche 6 bis 11, dadurch gekennzeichnet, daß die elektronische Schaltung (1) eine Schaltung zur Amplitudenbegrenzung des Einschaltsignals des Sprachaufnehmers (S) umfaßt.

13. Sprechhilfe nach einem der vorhergehenden Ansprüche, dadurch gekennzeichnet, daß der Geräuschgeber und/oder der Sprachaufnehmer (S) ein Körperschallaufnehmer bzw. ein Körperschallgeber sind.

14. Sprechhilfe nach einem der vorhergehenden Ansprüche, dadurch gekennzeichnet, daß der Geräuschgeber und/oder der Sprachaufnehmer (S) ein akustischer Luftschallaufnehmer bzw. akustischer Luftschallgeber sind.

15. Sprechhilfe nach einem der vorhergehenden Ansprüche, dadurch gekennzeichnet, daß sie einen elektrodynamischen Wandler (W) umfaßt, der sowohl als Geräuschgeber als auch als Sprachaufnehmer (S) ausgebildet ist.

16. Sprechhilfe nach Anspruch 15, dadurch gekennzeichnet, daß die elektronische Schaltung (1) derart ausgebildet ist, daß der elektrodynamische Wandler (W) in dem Betriebszustand als Sprachaufnehmer (S) von Gleich-und/oder Wechselspannungssignalen, die im Betriebszustand als Geräuschgeber an den elektrodynamischen Wandler (W) angelegt werden, im wesentlichen freigeschaltet wird.

17. Sprechhilfe nach Anspruch 16, dadurch gekennzeichnet, daß das Freischalten des elektrodynmamischen Wandlers (W) mittels eines in seine Zuleitung geschalteten Transistors (T₄) erfolgt, der in dem Betriebszustand als Geräuschgeber von der Triggerschaltung (3) oder dem das Maskierungsgeräusch erzeugenden Tongenerator (4) durchgeschaltet wird und in dem Betriebszustand als Sprachaufnehmer (S) gesperrt ist.

18. Sprechhilfe nach Anspruch 16, dadurch gekennzeichnet, daß das Freischalten des elektrodynmamischen Wandlers (W) mittels einer oder mittels zweier antiparallel in seine Zuleitung geschalteter Dioden (5) erfolgt, die in dem Betriebszustand als Geräuschgeber leitend ist bzw. sind und die in dem Betriebszustand als Sprachaufnehmer (S) gesperrt ist bzw. sind.

19. Sprechhilfe nach einem der vorhergehenden Ansprüche, dadurch gekennzeichnet, daß sie ein in ein Ohr (6, 7) einsetzbares Ohrstück (8, 9) umfaßt, das einen Sprachaufnehmer umfaßt.

20. Sprechhilfe nach Anspruch 19, dadurch gekennzeichnet, daß das Ohrstück (9) auch einen Geräuschgeber (W_{R}) aufweist.

21. Sprechhilfe nach Anspruch 19 oder 20, dadurch gekennzeichnet, daß das Ohrstück (9) derart ausgebildet ist, daß es den Gehörgang schalldicht oder schallreduzierend abschließt, um den Sprachaufnehmer (W_{R}) gegen Umgebungsschall abzuschirmen.

22. Sprechhilfe nach einem der Ansprüche 19 bis 21, dadurch gekennzeichnet, daß sie zwei in die Ohren (6, 7) einsetzbare Ohrstücke (8, 9) umfaßt, wobei in dem ersten Ohrstück (9) ein Sprachaufnehmer (W_{R}) und ein Geräuschgeber (W_{R}) und in dem zweiten Ohrstück (8) ein weiterer Geräuschgeber (W_{L}) angeordnet sind.

23. Sprechhilfe nach Anspruch 22, dadurch gekennzeichnet, daß in dem zweiten Ohrstück (8) kein Sprachaufnehmer (S) angeordnet ist.

24. Sprechhilfe nach Anspruch 22 oder 23, dadurch gekennzeichnet, daß das erste Ohrstück (9) nach Anspruch 20 ausgebildet ist und das zweite Ohrstück (8) einen den Umgebungsschall durchlassenden Kanal (11) aufweist.

25. Sprechhilfe nach einem der vorhergehenden Ansprüche, dadurch gekennzeichnet, daß sie zwei Geräuschgeber (W_{L}, W_{R}) umfaßt, die in je einem Ohr (6, 7) eines Benutzers angeordnet werden können.

26. Sprechhilfe nach einem der vorhergehenden Ansprüche, dadurch gekennzeichnet, daß sie nur einen Sprachaufnehmer (S) umfaßt.
